**Europäisches Patentamt**

**European Patent Office**

**Office européen des brevets**

(19)

(11) Numéro de publication: **0 174 228**

**A1**

(12)

# DEMANDE DE BREVET EUROPEEN

(21) Numéro de dépôt: **85401556.7**

(22) Date de dépôt: **30.07.85**

(51) Int. Cl.⁴: **G 01 N 33/569**
**G 01 N 33/577, C 12 N 7/00**
**C 07 K 15/00, C 12 P 21/00**
**C 12 N 5/00, C 12 N 15/00**
**//C12P21:00, C12R1:91**

(30) Priorité: **01.08.84 FR 8412226**

(43) Date de publication de la demande:
**12.03.86 Bulletin 86/11**

(84) Etats contractants désignés:
**AT BE CH DE FR GB IT LI LU NL SE**

(71) Demandeur: **INSTITUT PASTEUR**
**25-28, rue du Docteur Roux**
**F-75724 Paris Cedex 15(FR)**

(71) Demandeur: **INSTITUT NATIONAL DE LA SANTE ET DE**
**LA RECHERCHE MEDICALE (INSERM)**
**101, rue de Tolbiac**
**F-75654 Paris Cedex 13(FR)**

(71) Demandeur: **CENTRE NATIONAL DE LA RECHERCHE**
**SCIENTIFIQUE (C.N.R.S.)**
**15, Quai Anatole France**
**F-75007 Paris(FR)**

(72) Inventeur: **Orth, Gérard**
**49 Rue du Dr. Roux**
**F-92330 Sceaux(FR)**

(72) Inventeur: **Peries, Georges**
**50 Quai de Jemmapes**
**F-75010 Paris(FR)**

(72) Inventeur: **Pothier, Pierre**
**8 Rue Nicolas Rollin**
**F-21800 Chevigny St Sauveur(FR)**

(72) Inventeur: **Roseto, Alberto**
**34 Avenue Guy de Maupassant**
**F-78400 Chatou(FR)**

(72) Inventeur: **Guillemin, Marie-Claude**
**34 Avenue Guy de Maupassant**
**F-78400 Chatou(FR)**

(72) Inventeur: **Breitburd, Françoise**
**36 Rue Molitor**
**F-75016 Paris(FR)**

(74) Mandataire: **Phélip, Bruno et al,**
**c/o Cabinet Harlé & Phélip 21, rue de la Rochefoucauld**
**F-75009 Paris(FR)**

(54) **Procédé de diagnostic des infections à papillomavirus humains, anticorps monoclonaux spécifiques d'un type de papillomavirus humains, et lignées d'hybridomes les sécrétant.**

(57) "Procédé de diagnostic des infections à papillomavirus humains, anticorps monoclonaux spécifiques d'un type de papillomavirus humains, et lignées d'hypridomes les sécrétant."

Invention de: ORTH Gérard
PERIES Georges
POTHIER Pierre
ROSETO Alberto
GUILLEMIN Marie-Claude
BREITBURD Françoise
INSTITUT PASTEUR
INSTITUT NATIONAL DE LA SANTE ET DE LA RECHERCHE
MEDICALE (INSERM)
CENTRE NATIONAL DE LA RECHERCHE SCIENTIFIQUE
La présente invention concerne un procédé de diagnostic des infections à papillomavirus humains, les anticorps monoclonaux spécifiques de chaque type de papillomavirus humain et les lignées de cellules hybrides qui sécrètent ces anticorps spécifiques. Les hybridomes résultent de la fusion de cellules de myélome murin et de cellules de rates de souris ayant été immunisées contre ce virus.

Ces anticorps sont utilisés pour l'identification spécifique, le dosage et la purification des papillomavirus humains.
PAS DE DESSIN.

EP 0 174 228 A1

Croydon Printing Company Ltd.

La présente invention concerne un procédé de diagnostic des infections à papillomavirus humains, mettant en oeuvre des anticorps monoclonaux permettant de distinguer les papillomavirus bénins des papillomavirus potentiellement oncogènes.

Les méthodes classiques de réaction immunologique antigène/anticorps ne peuvent être systématiquement utilisées pour détecter la présence de certains types de papillomavirus humains en raison de la trop faible quantité disponible de papillomavirus, ce qui interdit l'immunisation d'animaux en vue de la préparation d'immun-sérums polyclonaux. Il est donc apparu nécessaire de mettre au point un procédé d'identification différentielle permettant de distinguer les papillomavirus humains bénins (HPV), le type 1 par exemple, des papillomavirus de type 16 ou 18 dont on connaît le potentiel oncogène (se référer à l'article de Lionel Crawford, Nature, 1984, volume 310 p.16).

Pour mettre en oeuvre le procédé selon l'invention, des anticorps monoclonaux sont préparés, à partir de lignées d'hybridomes spécifiques de chaque type de papillomavirus humain. A titre d'exemple de mise en oeuvre de l'invention, des anticorps monoclonaux, dirigés contre le papillomavirus humain de type 1 et des lignées de cellules hybrides qui sécrètent ces anticorps, ont été préparés.

La présente invention concerne un procédé

8178 EU

permettant la détection, la différenciation ou le dosage des papillomavirus humain (HPV) mettant en oeuvre des anticorps monoclonaux dirigés contre un type de papillomavirus humain, les lignées de cellules hybrides qui sécrètent ces anticorps et les anticorps monoclonaux spécifiques. Le procédé de diagnostic consiste à faire une recherche d'immunoréaction entre les anticorps monoclonaux de l'invention et un échantillon susceptible d'être infecté par le virus, tel qu'une coupe de biopsie ou un extrait d'une lésion. On peut utiliser un seul type d'anticorps ou de préférence un mélange pour mieux caractériser le papillomavirus en cause.

Les papillomavirus, de la famille des papovavirus provoquent, chez l'homme, des tumeurs épithéliales bénignes de la peau et des muqueuses, les verrues et condylomes, qui peuvent parfois dégénérer en tumeurs malignes. On a, jusqu'à présent, dénombré vingt-huit types de papillomavirus humains, en tenant compte des taux d'hybridation croisée des ADN inférieurs à 50 % ; récemment l'apparition de cancers génitaux et cutanés a été associée à la présence de certains types de ces virus, le type 1, entre autres, étant considéré comme parfaitement bénin.

Si l'on a pu montrer l'existence d'antigènes du groupe des papillomavirus et celle d'antigènes spécifiques des différents types, des anticorps polyclonaux spécifiques de la plupart de ces différents types de virus n'ont jamais été préparés notamment en raison de la très faible quantité de matériel antigénique disponible. Il est pourtant particulièrement souhaitable d'avoir des anticorps qui soient spécifiques, c'est-à-dire ne reconnaissent qu'un type de papillomavirus, pour préparer des réactifs d'identification, de différenciation, de dosage et de purification de ces virus, dans la mesure où certains types seulement semblent associés à des phénomènes de cancérogénèse. En outre, ces anticorps permettraient l'étude de la structure antigénique de ces virus.

Ainsi, la mise au point d'un réactif permettant de différencier un virus bénin, tel celui de type 1, des virus potentiellement oncogènes, comme ceux de type 16 ou 18, était particulièrement souhaitable, et c'est l'un des avantages fondamentaux de l'invention de permettre cette différenciation.

Dans EP-A-0 092 456, on décrit la structure de l'un des brins du génome de certains papillomavirus, et notamment celui humain de type 1, et on revendique les séquences d'ADN qui y sont comprises et qui codent pour des polypeptides contenant au moins un déterminant antigénique des papillomavirus, ainsi que les polypeptides correspondants. Rien dans ce document ne permet de faciliter l'identification de tels polypeptides; cette identification supposerait la préparation de tous les polypeptides pouvant découler de la séquence d'ADN décrite, et l'étude de leur éventuel pouvoir immunogène ; il n'y a par ailleurs aucune preuve formelle de ce que les peptides spécifiquement cités page 5, ligne 37 ou page 6, ligne 9, correspondent effectivement à un antigène de surface du virus et que ledit antigène soit spécifique d'un seul type de virus;

il semble donc possible de dire qu'il n'existait pas encore de moyen de détection des différents types de papillomavirus mettant en oeuvre une réaction immunologique, à la date de l'invention.

On sait, depuis le travail de Kohler et Milstein publié en 1975 dans Nature 256 p. 495, fabriquer des hybridomes, lignées cellulaires immortelles, par fusion de cellules de myélome de souris et de cellules de rate de souris. Lorsque ces dernières ont été préalablement immunisées contre un antigène donné, les hybridomes sécrèteront des anticorps dirigés contre cet antigène et ces anticorps seront monoclonaux du fait de la technique de sélection et de culture utilisée.

Si cette méthode a été largement appliquée depuis cette date, par exemple à l'isolement d'anticorps antiviraux comme décrit dans US 4 196 265, il n'était pas pour autant évident de pouvoir sélectionner des lignées de cellules qui sécrèteraient des anticorps spécifiques contre un type donné de papillomavirus, c'est-à-dire ne donnant pas ou peu de réaction croisée avec les autres types.

Les hybridomes, objets de l'invention, sont des sécréteurs des anticorps spécifiques d'un type particulier de papillomavirus humain et les anticorps monoclonaux ainsi obtenus sont un autre objet de l'invention. Le papillomavirus humain de type 1 (ou HPV1) convient particulièrement bien à l'étude des virus infectant l'homme.

On trouve des quantités importantes de ces particules virales dans les verrues plantaires profondes ; aussi des anticorps monoclonaux dirigés contre le virus de type 1 et les hybridomes qui les sécrètent, illustrent la présente invention.

Pour préparer les hybridomes selon l'invention on effectue de façon classique la fusion de cellules d'un myélome de souris et de cellules extraites de la rate de souris préalablement immunisées contre le papillomavirus du type choisi.

Dans le cas de papillomavirus de type 1, les particules virales sont extraites des verrues plantaires profondes humaines, purifiées en vue de leur injection à des souris. On peut s'assurer que cette préparation virale ne comprend que des virus de type 1 en isolant l'ADN présent dans un échantillon et vérifiant que cet ADN donne les fragments connus pour être ceux du virus HPV1, par action d'endonucléases, par exemple Hind II et Hind III. La préparation virale est utilisée pour immuniser des souris par injection sous-cutanée ou intrapéritonéale. On fera plusieurs injections successives,à intervalle de plusieurs semaines. Lorsque les souris sont immunisées, elles sont tuées, et leurs rates prélevées ; les cellules de ces rates sont utilisées pour la fusion.

On peut faire appel à différentes lignées de cellules de myélome murin pour effectuer la fusion ; elles doivent permettre la sélection ultérieure des hybridomes formés par des méthodes connues en soi ; on préfère des cellules ne sécrétant pas d'immunoglobulines et la souche SP2/0, résistante à la 8-azaguanine convient parfaitement pour permettre la séparation des hybridomes des cellules non-fusionnées.

La fusion est réalisée par mise en contact des deux sortes de cellules en présence d'un agent de fusion qui peut être un polyéthylène glycol ou encore le virus de Sendai.

Après la fusion, l'ensemble des cellules, après dilution, est mis en culture dans un milieu sélectif, où seuls les hybridomes pourront se développer ; dans le cas des souches de myélome SP2/O, la présence dans le milieu de culture d'azasérine entraînera la disparition des cellules de rate n'ayant pas fusionné. On ne met en culture que quelques cellules par puits d'une plaque et après quelques jours, de 5 à 15, lorsque le développement des cellules est devenu apparent, on recherche la présence, dans le surnageant des différents puits, d'anticorps anti-HPV1, par une méthode classique (immunofluorescence, immunodiffusion ou par méthode immunoenzymatique). On peut, par exemple, mettre en évidence les anticorps formés par leur réaction avec les antigènes de HPV1 présents dans des coupes de verrues plantaires congelées, réaction révélée par immunofluorescence ou par une technique immunoenzymatique ; par cette méthode, on détectera les anticorps spécifiques d'un type ou du groupe des papillomavirus. Par immunodiffusion, en utilisant les particules virales entières comme antigènes, on mettra uniquement en évidence les antigènes spécifiques d'un type de virus. On peut également détecter les anticorps monoclonaux par la méthode de type ELISA (enzyme linked immunosorbent assay), en utilisant les particules virales intactes comme antigènes.

Les cellules sécrétant des anticorps intéressants, ainsi mises en évidence, sont alors clonées par la technique de la dilution limite pour obtenir des cultures monoclonales.

Les anticorps monoclonaux sécrétés par ces clones de cellules peuvent être isolés dans les surnageants de leurs cultures, ou dans les liquides des ascites qui se forment après injection intrapéritonéale des hybridomes à des souris.

L'identification de la classe à laquelle appartiennent les immunoglobulines sécrétées par les hybridomes peut être effectuée par immunodiffusion selon une méthode connue, en utilisant des anti-anticorps de souris dirigés contre des immunoglobulines de souris (anti-$IgG_1$, anti-$IgG_2a$, anti-$IgG_2b$, anti-$IgG_3$).

Deux lignées d'hybridomes, objets de l'invention ont été déposées dans la Collection Nationale de micro-organismes de l'Institut Pasteur (CNCM) le 30 juillet 1984, sous les numéros I-324 et I-325.

Le clone n° I-324 porte la référence 334 B6, alors que le clone n°I-325 porte la référence 405 D5. Ces hybridomes qui résultent de la fusion de cellules de myélome SP 2/0 et de cellules de souris Balb/c, ont sécrété depuis plus d'un an des anticorps monoclonaux dirigés spécifiquement contre le virus HPV1 ; en effet, ces anticorps dilués au 1/400 (v/v), permettent de déceler la présence d'antigènes de HPV 1 dans des coupes de verrues plantaires congelées, alors que, dilués au 1/10 (v/v), ils ne donnent aucune réaction décelable par immunofluorescence ou méthode immunoenzymatique (révélation de l'activité peroxydasique) dans des coupes congelées de verrues dues aux papillomavirus de type 2, 3, 4, 5, 6 et 7.

Dans le cas des papillomavirus humains des autres types, dans la mesure où ceux-ci ne seraient disponibles qu'en faible quantité, par exemple parce qu'on ne sait pas les cultiver, on pourra préparer des anticorps spécifiques, en quantité suffisante pour leur application à la préparation des anticorps monoclonaux selon l'invention en utilisant des techniques d'hyper-immunisation des animaux, comme par exemple celle décrite par LANGE et Coll. dans J. of Immuno-logical Methods (1983) vol. 63 p.123-131 et qui consiste à coupler l'antigène viral à un lipopoly-saccharide. On pourra aussi les obtenir à partir de fragments d'ADN spécifiques d'un type de papillomavirus, clonés par des méthodes classiques; Certains des ces fragments sont décrits dans J. Virol (1984) 52 1013-1018 de Kremsdorf D. et Coll. "Molecular cloning and characterization of the genomes of nine newly recognized HPV types associated with epidermodysplasia verruciformis".

Les anticorps monoclonaux selon l'invention spécifiques d'un type de virus, sont avantageusement utilisés pour préparer des réactifs immunologiques de détection, d'identification, de dosage et de purification du type de virus correspondant par des méthodes dont le principe est connu en soi. L'application de ces anticorps monoclonaux à la préparation de ces réactifs immunologiques, est un autre objet de l'invention. Les réactifs d'identification de l'HPV1 contenant les anticorps monoclonaux spécifiques de l'HPV1 sont utilisés pour la détermination de la gravité des infections à papillomavirus, c'est-à-dire pour distinguer une infection considérée comme banale (à HPV1) d'une infection potentiellement oncogène.

9

Le procédé de diagnostic consiste à faire un essai immunologique, dont le principe de la mise en oeuvre est connu, entre des particules virales ou des antigènes viraux présents dans la lésion avec les anticorps monoclonaux selon l'invention spécifiques du type HPV1 ou de tout autre type de papillomavirus humain dont l'activité ou l'absence d'activité oncogène est connue.

Dans ce qui suit, on illustre les procédés mis en oeuvre pour préparer les hybridomes et les anticorps monoclonaux, selon l'invention.

EXEMPLE

a) Immunisation des animaux.

Les particules virales HPV1 ont été isolées et purfiées selon la méthode décrite dans J. Virol 24 108-120 (1977).

On a injecté par voie sous-cutanée, à trois souris de souche Balb/c, une suspension de ces particules dans un tampon phosphate salin, mélangée à un volume égal d'adjuvant complet de Freund; chaque injection correspondait à 50 µg de protéines virales. L'injection a été répétée après 2 semaines ; puis un mois après la première injection, la même quantité de particules virales a été injectée, par voie intrapéritonéale, aux animaux, sans adjuvant ; les souris ont été tuées trois jours après cette dernière injection et leurs rates prélevées.

b) Fusion

Les cellules des rates, environ $2 \times 10^8$ cellules par animal, après isolement et lavage avec du milieu RPMI 1640, supplémenté par 20 % de sérum de veau foetal, ont été fusionnées avec des cellules de myélome de souris de type SP 2/0. On a mis pour cela en contact environ une cellule de myélome pour 10 cellules de rate, en présence de 1 ml de polyéthylène glycol à 50 % pendant 1 minute 30, à 37°C. Les hybridomes ont ensuite été séparés des cellules non fusionnées par culture sur un milieu classique composé de RPMI 1640, supplémenté par 20 % de sérum de veau foetal, par de l'hypoxanthine ($10^{-5}$M) et de l'azasérine ($5 \times 10^{-5}$M). Le milieu RPMI couram-ment utilisé pour les cultures de cellules est commercialisé par GIBCO - P.O. Box 35 - Paisley - Scotland. On a déposé environ $5 \times 10^5$ cellules par puits de culture.

c) Recherche des hybridomes sécréteurs

Les cellules fusionnées avaient été réparties dans des puits et 12 jours après le début de la culture, on a déterminé si les surnageants de chacun de ces puits contenaient des anticorps anti-HPV1, par les méthodes précédemment citées dans J. Virology (1977) 24 p.108-120 et Virology (1978) 91 p. 243-255.

On a trouvé deux puits dans lesquels les surnageants donnaient un résultat positif par la méthode d'immunofluorescence ou à l'immuno-peroxydase (sur coupes congelées de verrues) et par la méthode par immunodiffusion (contre des particules virales entières). Ces deux puits contenaient donc des anticorps spécifiques d'HPV1.

d) Clonage

Les cultures correspondantes dénommées B6 et D5 ont été clonées par dilution limite, selon un principe connu, de telle sorte que les cultures dans chaque puits ne dérivent plus que d'une cellule. Les surnageants de ces puits ont été étudiés, lorsque la croissance y était suffisante, pour ne conserver que les puits où les anticorps monoclonaux étaient présents. On a ainsi isolé deux clones, 334 B6 et 405 D5.

e) Identification des polypeptides viraux portant les déterminants antigéniques reconnus par les anticorps monoclonaux secrétés par les clones 334 B6 et 405 D5.

On sait que les particules virales sont dissociées à 100°C par action d'un détergent tel que le SDS (sodium dodécyl-sulfate) (conc. 3%), en présence d'urée 4M et de mercapto-2 éthanol (concentration finale 5 %), en trois sortes de polypeptides séparables par électrophorèse en gel de polyacrylamide ; l'un en grande quantité, de masse moléculaire apparente 54 000 daltons, un second, en plus faible quantité, de masse 76 000 daltons et d'autres de masses comprises entre 12 500 et 16 500 daltons, comme décrit dans les deux articles précédemment cités.

Les anticorps monoclonaux sécrétés par les cellules de lignée 334 B6 et 405 D5 réagissent avec les polypeptides de masse 76 000 daltons et de masse 54 000 daltons, qui sont donc tous deux porteurs de déterminants antigéniques du virus. Ceci a été mis en évidence par la technique d'immunoréplique, dite Western Blot.

Les anticorps monoclonaux sont sécrétés par les cellules en culture en quantité importante: les titres des cultures, qui sont l'inverse de la plus grande dilution de surnageant révélant les noyaux infectés dans des coupes de verrues congelées ou donnant une ligne de précipitation avec des particules virales, sont compris entre 10 et 100 dans le cas de l'immunofluorescence ou de la techique d'immunoperoxydase et entre 1 et 16 pour la technique d'immunodiffusion.

Les liquides d'ascites résultant de l'injection intrapéritonéale aux souris Balb/c de $2 \times 10^6$ hybridomes ont un titre, 12 jours après l'injection, allant de 200 à 400 (détermination par immunofluorescence) ou de 200 à 400 (détermination par technique d'immunoperoxydase) ou de 50 à 200 (détermination par immunodiffusion). Les classes auxquelles ces anticorps appartiennent ont été déterminées par immunodiffusion ; ce sont la sous-classe $IgG_1$ (souche 334 B6) et la sous-classe $IgG_{2a}$ (souche 405 D5).

REVENDICATIONS

1. Procédé de détection, de différenciation ou de dosage des papillomavirus humains dans une lésion, caractérisé en ce que l'on fait une recherche d'immunoréaction avec des anticorps monoclonaux spécifiques de certains papillomavirus.

2. Procédé de purification de particules virales ou d'antigènes des papillomavirus humains, caractérisé en ce qu'il met en oeuvre des anticorps monoclonaux spécifiques d'un type de papillomavirus.

3. Procédé selon l'une des revendications 1 ou 2, caractérisé en ce que le papillomavirus est du type 1 (HPV1).

4. Anticorps monoclonal, caractérisé en ce qu'il est dirigé contre un seul type de papillomavirus humain.

5. Anticorps monoclonal selon la revendication 4, caractérisé en ce qu'il est dirigé contre les papillomavirus de type 1.

6. Anticorps monoclonal selon la revendication 5, caractérisé en ce qu'il a les caractéristiques immunologiques de ceux sécrétés par la lignée d'hybridomes dont un échantillon est déposé à la CNCM sous le ° I-324.

7. Anticorps monoclonal selon la revendication 5, caractérisé en ce qu'il a les caractéristiques immunologiques de ceux sécrétés par la lignée d'hybridomes dont un échantillon est déposé à la CNCM sous le n° I-325.

8. Cellules hybrides appartenant à la lignée de cellules dont des échantillons sont déposés à la CNCM sous le n° I-324.

9. Cellules hybrides appartenant à la lignée de cellules dont des échantillons sont déposés à la CNCM sous le n° I-325.

Office européen des brevets

**0174228**
Numéro de la demande

# RAPPORT DE RECHERCHE EUROPEENNE

EP 85 40 1556

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| D,Y | EP-A-0 092 456 (INSTITUT PASTEUR) <br> * Revendications 1,8,11,12; page 12, lignes 27-34; page 14, lignes 1-7 * | 1-9 | |
| D,Y | US-A-4 196 265 (H. KOPRUWSKI et al.) <br> * Revendications 1-10; colonne 2, lignes 6-9; colonne 9, lignes 40-50 * | 1-9 | |
| P,X | JOURNAL OF GENERAL VIROLOGY, vol. 65, no. 8, 1984, pages 1319-1324, SGM, US; A. ROSETO et al.: "Monoclonal antibodies to the major capsid protein of human papillomavirus type 1" <br> * En entier * | 1-9 | **DOMAINES TECHNIQUES RECHERCHES (Int Cl 4)** <br><br> C 12 P <br> A 61 K <br> G 01 N <br> C 12 N |

Le présent rapport de recherche a été établi pour toutes les revendications

| Lieu de la recherche | Date d'achèvement de la recherche | Examinateur |
|---|---|---|
| LA HAYE | 08-11-1985 | RYCKEBOSCH A.O.A. |